# EUROPÄISCHE PATENTSCHRIFT

(11) **EP 2 420 273 B1**
(45) Veröffentlichungstag und Bekanntmachung des Hinweises auf die Patenterteilung: **03.04.2013**
(21) Anmeldenummer: 11006786.5
(22) Anmeldetag: 19.08.2011
(51) Int. Cl.: A61M 5/142

(54) **Auffüllset für implantierbare Infusionspumpen**
Filling set for implantable infusion pumps
Ensemble de remplissage pour pompes à perfusion implantables

(30) Priorität: 21.08.2010 DE 202010011663 U
(43) Veröffentlichungstag der Anmeldung: 22.02.2012
(73) Patentinhaber: Medizintechnik Promedt GmbH, 25436 Tornesch (DE)
(72) Erfinder: Tiedemann, Lothar, 22926 Ahrensburg (DE)
(74) Vertreter: Lobemeier, Martin Landolf

(56) Entgegenhaltungen:
- EP-A1- 0 433 485
- EP-A1- 1 527 794
- WO-A1-93/00944
- DE-A1- 10 061 818
- US-A- 5 176 658
- US-B1- 6 261 282

## Beschreibung

Die Erfindung betrifft implantierte Medikamentenpumpen zur Schmerz- oder Spastiktherapie mit den Merkmalen des Oberbegriffs des Anspruchs 1, die eine Infusionslösung den Körper eines Patienten applizieren und nach deren Abgabe wieder befüllt werden müssen.

Vor jeder Wiederbefüllung müssen die implantierten Infusionspumpen zunächst entleert werden, um sicherzustellen, dass das vollständige Reservoirvolumen zur Wiederbefüllung zur Verfügung steht. Der Zugang zur implantierten Medikamentenpumpe erfolgt über ein Auffüllseptum, das in der Regel aus Silikon gefertigt wird und speziellen Auffülkanülen, die in Auffüllsets der verschiedenen Hersteller enthalten sind.

Zum Entleeren der implantierten Infusionspumpen werden die Auffüllsepten mit einer speziellen Kanüle punktiert. Die restliche Medikamentenlösung aus den implantierten Infusionspumpen wird über einfache Schlauchleitungen abgeleitet, an deren Ende ein leerer Spritzenzylinder zum Auffangen des Restmedikamentes konnektiert wird.

Nach der Entleerung wird das Restmedikament zusammen mit dem Spritzenzylinder verworfen. Bei einigen Einmalsets wird hierbei auch die Verbindungsleitung ebenfalls verworfen.

Zur Wiederbefüllung muss der Innendruck der implantierten Medikamentenpumpen (ca. 0,8 - 2,5 bar) überwunden werden. Aufgrund der physikalischen Verhältnisse eignen sich hierzu besonders Spritzen mit geringen Durchmessern (z. B., 10 ml Spritzen). In diese Spritzen kann die Medikamentenlösung direkt aufgezogen werden. Nachteil hierbei ist, dass man bei einem Füllvolumen von beispielsweise 40 ml vier einzelne Spritzen aufziehen muss. Daher befinden sich Systeme am Markt, die es ermöglichen, aus einer mit Medikamentenlösung aufgezogenen 60 ml Spritze die Infusionslösung zur Befüllung in eine 10 ml Spritze unter aus mikrobiologischer Sicht unzureichenden Bedingungen umzufüllen (vgl. EP 1 527 794 A1 und DE 100 61 818 A1).

Durch die notwendigen mehrfachen manuellen Manipulationen zum Zusammenfügen der Flüssigkeitswege sind die am Markt verfügbaren Auffüllsets hinsichtlich ihrer mikrobiologischen Kontaminationsgefahr nicht optimal. Selbst bei der Verwendung von Bakterienfiltern am Beginn des Befüllschlauches ist nicht sichergestellt, dass beim notwendigen Entfernen der Auffüllkanülen vom Entleerungsschlauch und anschließendem Verbinden mit dem Befüllschlauch störende Bakterien in den Kanülenansatz der Auffüllkanüle eingetragen werden.

Der Erfindung liegt damit die Aufgabe zugrunde, ein Auffüllset für implantierbare Infusionspumpen zu schaffen, bei dem das Schlauchsystem nicht nur bei dem Füllen, sondern auch bei dem Entleeren geschlossen und damit aseptisch bleibt.

Erfindungsgemäß wird diese Aufgabe gelöst durch ein in den Verbindungsschlauch ein oder an das Dreiwege-Rückschlagventil angesetzten T-Port, der bei Ansetzen einer Entleerungsspritze an das T-Port einen Weg von der Kanüle zu der Entleerungsspritze öffnet und der Weg von dem Dreiwege-Rückschlagventil zu der Entleerungsspritze gesperrt wird.

Vorzugsweise wird der T-Port von dem Luer-Anschlussstück der Entleerungsspritze aktiviert.

Die Erfindung wird im Folgenden anhand einer Zeichnung erläutert.

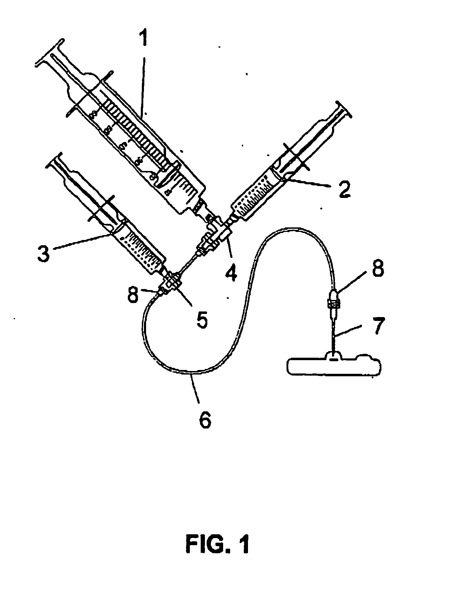

Dabei zeigt die einzige Figur eine schematische Darstellung des Auffüllsets.

Das Auffüllset für implantierbare Infusionspumpen besteht aus einer Vorratsspritze 1 für die in eine implantierbare Infusionspumpe einzubringende Medikamentenlösung sowie einer mit dieser über ein Dreiwege-Rückschlagventil 4 verbundene Befüllspritze 2. Weiter ist ein von dem Dreiwege-Rückschlagventil 4 durch das Septum der Infusionspumpe zu deren Befüllung einzustechenden Kanüle führender Verbindungsschlauch 6 vorgesehen.

In den Verbindungsschlauch 6 ein- oder an das Dreiwege-Rückschlagventil 4 ist ein T-Port 5 (oder ein anderer Dreiwege-Port) angesetzt, der im senkrechten T-Abgang eine abdichtende Gummilippe aufweist, die beim Aufschrauben eines Luerlocks durch den inneren Luerkonus aufgestoßen wird. Damit beim Entleeren keine Flüssigkeit in die Vorratsspritze und Befullspitze aufsteigt, blockiert das 3-Wegeventil mit seinem Rückschlagventil den Durchfluss.

An das T-Port 5 ist eine Entleerungsspritze 3 angesetzt.

Die Verwendung dieses Auffüllsets ist folgendermaßen: Das Auffüllseptum der Infusionspumpe wird mittels der Kanüle 7 punktiert. Zur Entleerung der Infusionspumpe wird die Entleerungsspritze 3 an den von dem Luer-Anschluss der Entleerungsspritze 3 aktivierten, also geöffneten Entleerungsport 5 angeschlossen. Im Moment der vollständigen Konnektion mit der Spritze öffnet der von dem Luer aktivierte T-Port 5 selbsttätig. Durch den Überdruck der Infusionspumpe entleert sich das Restmedikament bei korrekter Punktion des Auffüllseptums selbständig in die Entleerungsspritze 3. Die Entleerungsspritze 3 wird nach vollständiger Entleerung diskonnektiert. Der von dem Luer-Anschluss aktiviert gewesene T-Port 5 schließt nun selbständig bei Entfernen der Spritze.

Nach Abschluss der Entleerung wird eine mit Medikamentenlösung gefüllte Vorratsspritze 1 an das Dreiwege-Rückschlagventil 4 angeschlossen. Eine leere Befüllungsspritze 2 wird an den zweiten Anschluss des Dreiwege-Rückschlagventils 4 angeschlossen. Zur Befüllung wird aus der Vorratsspritze 1 die benötigte Medikamentenmenge in die Befüllspritze 2 umgefüllt. Das so umgefüllte Medikament wird nun mit der Befüllspritze 2 über die Kanüle 7 in die implantierte Infusionspumpe eingeführt. Sodann werden weitere Befüllvorgänge aus der Vorratsspritze 1 in die Befüllspritze 2 durchgeführt, bis die verschriebene Medikamentenlösung in die Infusionspumpe appliziert ist.

Nach dem Befüllen der Infusionspumpe wird die Auffüllkanüle 7 aus dem Auffüllseptum entfernt und das Schlauchsystem wird mit den zugehörigen Spritzen entsorgt.

## Patentansprüche

1. Auffüllset für implantierte Infusionspumpen, mit einer Vorratsspritze (1) für die Medikamentenlösung, einer mit dieser über ein Dreiwege-Rückschlagventil (4) verbundene Befüllspritze (2) und einem von dem Dreiwege-Rückschlagventil (4) zu einem durch das Septum der Infusionspumpe zu deren Befüllung einzustechenden Kanüle (7) führenden Verbindungsschlauch (6), **gekennzeichnet durch** ein in den Verbindungsschlauch (6) ein- oder an das Dreiwege-Rückschlagventil (4) angesetzten T-Port (5), der bei Ansetzen einer Entleerungsspritze (3) an den T-Port (5) einen Weg von der Kanüle (7) zu der Entleerungsspritze (3) öffnet

2. Auffüllset nach Anspruch 1, **dadurch gekennzeichnet, dass** der T-Port (5) von einem Luer-Anschlussstück der Entleerungsspritze (3) aktiviert wird.

## Claims

1. A replenishing set for implanted infusion pumps, having a supply syringe (1) for the medicine solution, a filling syringe (2) connected thereto by a three-way check valve (4), and a connection hose (6) that is guided from the three-way check valve (4) to a cannula (7) that is to be inserted through the septum of the infusion pump for the purpose of filling it, **characterized by** a T-port (5) that is inserted into the connecting hose (6) or attached to the three-way check valve (4), that, when an emptying syringe (3) is coupled to the T-port (5), opens a path from the cannula (7) to the emptying syringe (3).

2. The replenishing set according to Claim 1, **characterized in that** the T-port (5) is activated by a Luer connecting piece of the emptying syringe (3).

## Revendications

1. Kit de remplissage pour pompes d'infusion implantées, avec une seringue de réserve (1) pour la solution de médicaments, une seringue de remplissage (2), reliée avec elle via une valve anti-retour à trois voies (4), et un tuyau d'accouplement (6), qui va de la valve anti-retour à trois voies (4) vers une canule (7), à enfoncer à travers la cloison de la pompe d'infusion pour son ravitaillement, **caractérisé par** un port en T (5), embouti sur la valve anti-retour à trois voies (4), qui, lors du contact avec la seringue de vidange (3) au port en T (5), ouvre une voie de la canule (7) vers la seringue de vidange (3).

2. Kit de remplissage d'après revendication 1, **caractérisé en ce que** le port en T (5) est activé par une pièce de raccordement Luer de la seringue de vidange (3).
